# EUROPEAN PATENT SPECIFICATION

(11) **EP 1 613 775 B1**
(45) Date of publication and mention of the grant of the patent: **29.08.2012**
(21) Application number: 04758378.6
(22) Date of filing: 25.03.2004
(51) Int. Cl.: C12N 15/117, A61K 31/7105, A61K 31/713, A61P 35/02

(54) **SELECTED RNA MOTIFS IN ANTI-LEUKEMIA THERAPY**
AUSGEWÄHLTE RNA-MOTIVE ZUR ANTI-LEUKÄMIE BEHANDLUNG
MOTIFS D'ARN SÉLECTIONNÉS POUR LA THÉRAPIE ANTI-LEUCÉMIE

(30) Priority: 26.03.2003 US 457745 P; 26.08.2003 US 497784 P
(43) Date of publication of application: 11.01.2006
(73) Proprietor: Multicell Immunotherapeutics, Inc., San Diego, CA 92121 (US)
(72) Inventor: SMITH, Dan, San Diego, CA 92122 (US); BOT, Adrian, Valencia, CA 91354 (US)
(74) Representative: Stuart, Ian Alexander
(86) International application number: PCT/US2004/009261
(87) International publication number: WO 2004/087877

(56) References cited:
- WO-A2-03/078595
- US-A- 3 906 092
- STARK G R; KERR I M; WILLIAMS B R; SILVERMAN R H; SCHREIBER R D: "How cells respond to interferons." 1998, ANNUAL REVIEW OF BIOCHEMISTRY 1998, VOL. 67, PAGE(S) 227 - 264 , XP002543752 ISSN: 0066-4154 * page 242 * * page 245 - page 247 * * page 255 - page 256 *
- SCARIM ANNA L ET AL.: "Mechanisms of beta-cell death in response to double-stranded (ds) RNA and interferon-gamma: dsRNA-dependent protein kinase apoptosis and nitric oxide-dependent necrosis" AMERICAN JOURNAL OF PATHOLOGY, vol. 159, no. 1, July 2001 (2001-07), pages 273-283, XP002543753 ISSN: 0002-9440
- WANG L; ET AL: "Noncoding RNA danger motifs bridge innate and adaptive immunity and are potent adjuvants for vaccination" JOURNAL OF CLINICAL INVESTIGATION, vol. 110, no. 8, 1 October 2002 (2002-10-01), pages 1175-1184, XP002971606 ISSN: 0021-9738
- LACOUR J; ET AL: "POLYADENYLIC-POLYURIDYLIC ACID AS ADJUVANT IN THE TREATMENT OF OPERABLE BREAST CANCER: RECENT RESULTS" EUROPEAN JOURNAL OF SURGICAL ONCOLOGY, vol. 14, no. 4, 1 August 1988 (1988-08-01) , pages 311-316, XP009056482 ISSN: 0748-7983
- HAINES D S; STRAUSS K I; GILLESPIE D H: "CELLULAR RESPONSE TO DOUBLE-STRANDED RNA" JOURNAL OF CELLULAR BIOCHEMISTRY, vol. 46, no. 1, 1 January 1991 (1991-01-01), pages 9-20, XP009010827 ISSN: 0730-2312
- DER SANDY D; YANG YI-LI; WEISSMANN CHARLES; WILLIAMS BRYAN R G: "A double-stranded RNA-activated protein kinase-dependent pathway mediating stress-induced apoptosis" PROCEEDINGS OF THE NATIONAL ACADEMY OF SCIENCES OF THE UNITED STATES OF AMERICA, vol. 94, no. 7, 1997, pages 3279-3283, XP002543754 ISSN: 0027-8424
- ELBASHIR ET AL: 'RNA interference is mediated by 21- and 22-nucleotide RNAs' GENES AND DEVELOPMENT vol. 15, 2000, pages 188 - 200, XP002204651
- WAINNY ET AL: 'Specific interference with gene function by double-stranded RNA in early mouse development' NATURE CELL BIOLOGY vol. 2, 2000, pages 70 - 75, XP002138445
- SVOBODA ET AL: 'Selective reduction of dormant maternal mRNAs in mouse oocytes by RNA interference' DEVELOPMENT vol. 127, 2000, pages 4147 - 4156, XP001064763
- ZELEZNICK ET AL: 'Treatment of leukemic (L-1210) Mice with Double-stranded Polyribonucleotides' PROC SOC EXP BIOL MED vol. 130, 1969, pages 4147 - 4156, XP008107166
- BRANCH A.D. ET AL: 'A good antisense molecule is hard to find' TIBS vol. 23, 1998, pages 45 - 50, XP004108000
- JEN ET AL: 'Suppression of gene expression by targeted disruption of messenger RNA: Available options and current strategies' STEM CELLS vol. 18, 2000, pages 307 - 319, XP002181426
- OATES ET AL: 'Too much interference: Injection of double-stranded RAN has nonspecific effects in the zebrafish embryo' DEVELOPMENTAL BIOLOGY vol. 224, 2000, pages 20 - 28, XP002945261
- COBURN ET AL: 'siRNAs: a new wave of RNA-based therapeutics' JOURNAL OF ANTIMICROBIAL CHEMOTHERAPY vol. 51, 2003, pages 753 - 756, XP002992788
- AGAMI ET AL: 'RNAi and related mechanisms and their potential use for therapy' CURRENT OPINION IN CHEMICAL BIOLOGY vol. 6, 2002, pages 829 - 834, XP002239988
- GREEN ET AL: 'Antisense Oligonucleotides: An evolving technology for the modulation of gene expression in human diseases' J AM COLL SURG vol. 191, 2000, pages 93 - 105, XP002948604
- AGRAWAL ET AL: 'Antisense therapeutics: is it as simple as complementary base recognition' MOLECULAR MEDICINE TODAY vol. 61, 2000, pages 72 - 80, XP000979511
- KHAN ET AL: "Polyadenylic-polyuridylic acid enhances the natural cell-mediated cytotoxicity in patients with breast cancer undergoing mastectomy", SURGERY, C.V. MOSBY CO., ST. LOUIS, US, vol. 118, no. 3, 1 September 1995 (1995-09-01), pages 531-538, XP005476239, ISSN: 0039-6060, DOI: 10.1016/S0039-6060(05)80370-8
- DRAKE W P ET AL: "PROPHYLACTIC THERAPY OF SPONTANEOUS LEUKEMIA IN AKR MICE BY POLY ADENYLIC-ACID POLY URIDYLIC-ACID", JOURNAL OF THE NATIONAL CANCER INSTITUTE, vol. 52, no. 3, 1974, pages 941-944, XP008149193, ISSN: 0027-8874

## Description

### Field of the Invention

The present application relates generally to low and high molecular weight double stranded RNA ("dsRNA") and single stranded RNA ("ssRNA") in their use to induce cell death in leukemia cells.

### Background of the Invention

Several investigators in the 1970s and 1980s have reported the binding of high molecular weight DNA to cell membranes. Various polynucleotide strands have been extensively evaluated as biological response modifiers. One example is polyl:polyC which is a potent inducer of IFN production as well as a macrophage activator and inducer of NK activity (Talmadge et. al., "Immunomodulatory effects in mice of polyinosinic-polycytidylic acid complexed with poly-L:-lysine and carboxymethylcellulose". Cancer Res. 45:1058, 1985.) Several clinical trials were conducted using polyI:polyC complexed with poly-L-lysine and carboxymethylcellulose to improve stability (Talmadge, J. et al., "Immunomodulatory Effects In Mice of Polyinosinic-polycytidylic Acid Complexed With Poly-L Lysine and Carboxymethylcellulose", Cancer Res. 45:1058, 1985). However, toxic side effects due to the cytokine TNF - alpha have prevented polyI:polyC from becoming a useful therapeutic agent.

Short interfering RNA (siRNA) have been shown to induce sequence-specific posttranscriptional gene silencing of homologous RNA using 21 and 22 nucleotide RNA (Elbashir et al "RNA interference is mediated by 21-22 nucleotide RNAs". Genes Dev. 2001 Jan 15;15(2):188-200) which is quite different than the non-sequence specific action of the dsRNA noted in this disclosure.

Certain DNA structures also have been reported to have the potential to activate lymphocytes. For example, nucleosomal protein-DNA complexes in spleen cell supernatants was reported to cause B cell proliferation and immunoglobulin secretion (Bell, et al. "Immunogenic DNA-related factors". J. Clin. Invest. 85:1487, 1990). Naked DNA has also been reported to have immune effects. For example, it was reported that 260 to 800 bp fragments of poly (dG):(dC) were mitogenic for B cells (Messina, et al. "The influence of DNA structure on the in vitro stimulation of murine lymphocytes by natural and synthetic polynucleotide antigens". Cell. Immunol. 147:148, 1993). Pisetsky et al. reported that pure mammalian DNA has no detectable immune effects, but that DNA from certain bacteria induces B cell activation and immunoglobulin secretion (Pisetsky et al. "Stimulation of in vitro murine lymphocyte proliferation by bacterial DNA". J. Immunol. 147:1759; 1991). There were also certain oligodeoxynucleotides containing unmethylated cytosine-guanine (CpG) dinucleotides activate lymphocytes as evidenced by *in vitro* and *in vivo* data.

Non-coding RNA motifs are produced by cells infected with negative stranded RNA or certain DNA viruses. Certain dsRNAs are recognized by innate immune cells and known to bind to Toll-like receptor 3 (Alexopoulou et al. "Recognition of doublestranded RNA and activation of NF-kappaB by Toll-like receptor 3". Nature 413:732-738; 2001). In addition, these dsRNAs have been found to influence both the innate and adaptive immune response (Wang et al. "Noncoding RNA danger motifs bridge innate and adaptive immunity and are potent adjuvants for vaccination". J. Clin. Invest. 110: 1175-84; 2002).

Stark et al (Annual Review of Biochemistry (1998) 67: 227-264) describe interferon induced antiviral pathways that utilise the dsRNA-dependent protein kinase (PKR), the 2-5A system, and the Mx proteins. Stark refers to work by Der et al (Proceedings of the National Academy of Sciences (1997) 94: 3279-3283) which demonstrates that dsRNAs (specifically pI:pC) induce the production of INFα/β, and is a pro-apoptotic agent.

Scarim et al (American Journal of Pathology 159(1): 273-283) showed that the synthetic dsRNA molecule polyinosinic-polycytidylic acid (pl:pC) stimulates β-cell DNA damage and apoptosis without impairing islet secretory function. In contrast, the combination of poly IC and interferon (IFN)γ stimulates DNA damage, apoptosis and necrosis of islet cells, and this damage is associated with the inhibition of glucose-stimulated insulin secretion.

Drake et al. (J.Natl. Cancer Inst. 52:941-944) showed the beneficial effects of polyadenylic-polyuridylic acid mixtures on leukemia in mice.

### Summary of the Invention

The present application is directed to the use of dsRNA and ssRNA for the purpose of inducing cell death in leukemia cells. Specifically, low molecular weight pA:pU and pA are shown to induce cell death in proliferating cells, to arrest proliferation of leukemia cells, to cause rapid induction of the pro-inflammatory cytokine TNF-alpha. Also shown herein, but not part of the invention is the use of high molecular weight RNA to induce production of IL-12 a modulator in the induction of IFN-gamma T cells and the Th1 immune response.

The present invention has practical application in anti-leukemia therapy in the arrest of proliferation of transformed leukemia cells, and to rapidly induce the cytokine TNF-alpha. This technology circumvents the need for a cellular transfection reagent which makes it compatible with *in vivo* applications and lacks the need for epitope or gene specificity, as with antisense or siRNAs, in therapy for solid cancers and leukemias. Antisense compounds need transfection reagents for both *in vitro* and *in vivo* uses and in general antisense compounds access few cells. In contrast, low molecular weight RNAs (single stranded and double stranded) transport easily across cell membranes and do not require transfection reagents.

Certain of the disclosed dsRNAs induce in innate cells either, or both, pro-inflammatory (TNF-alpha) responses or pro-apoptotic responses which may enhance the cross presentation of antigen and enhance specific immune effectors specifically toward a Th-1 response. Apoptotic cells are an excellent source of antigenic material and important for induction of effector cells (Th1 or Tc1) in cancer and infectious disease.

It is not entirely understood how low molecular'weight and high molecular weight RNA strands are inducing apoptosis and/or cell death in cells. The RNA motifs may be being recognized by either Toll-like receptors on the cell membrane or following cell internalization by cells, function by mediating negative regulation of gene expression causing non-specific gene silencing. Regarding the low molecular weight RNA (ssRNA and dsRNA), it is believed that they enter the cell directly without utilizing a cell receptor. Regarding high molecular weight RNA strands (ssRNA and dsRNA), it is believed that they enter into the cell via a cell receptor and that secondary structure is therefore important or possibly critical to their function.

This invention teaches that small or low molecular weight ssRNA or dsRNA induce rapid cell death with induction of TNF-alpha in a fast growing transformed cell. Also described herein, in fast growing transformed cells, large or high molecular weight ssRNA or dsRNA induce apoptosis with induction of IL-12, a known modulator in the induction of IFN-gamma T cells and of a Th-1 immune response, and TNF-alpha to a lesser extent, than found when induced by the low molecular weight ssRNA or dsRNA. Further, high molecular weight ssRNA or dsRNA can also be used an adjuvant and administered with an antigen to increase T₁ immunity and is important in use against infectious disease and cancers as taught in WO 03/078595 and PCT/US2003/030188. In contrast, low molecular weight RNAs (single or double stranded), can be used as adjuvants only in some cases.

It is believed the pro-apoptotic and/or cell necrotic cellular effects of the disclosed RNA compounds to be, to a great extent, more specific to cells of high or fast proliferation capacity (as is the case with many cancer and tumor cells) in contrast to primary cells. Regarding amounts or dosage to be therapeutically effective in humans, the RNA compositions of the present invention may be administered to human patients topically, systematically, or by direct injection into a tumor, in solutions or in emulsions, and in amounts in the ranges of 1 ng/kg - 999 mg/kg. Therapeutically effective dosages in humans range from 1 ng/kg - 10 ng/kg, to 10 ng/kg - 500 ng/kg, to 500 ng/kg - 999 ng/kg, 1µg/kg 10 µg/kg, to 10 µg/kg - 100 µg/kg, 100 µg/kg - 200 µg/kg, 200 µg/mg - 500 µg/mg, 500 µg/kg - 999 µg/kg, to 1mg/kg - 10 mg/kg, to 10 mg/kg - 100 mg/kg, to 100 mg/kg - 200 mg/kg and 200 mg/kg - 500 mg/kg. Most preferable human dosages, depending on the application, are in the range of 1- 999 µg/kg and most preferably 1 - 500 µg/kg or 1- 100 µg/kg. If injected directly into a tumor mass, it is expected that the dosages would be in the 1 mg/kg - 500 mg/kg and in the 1 mg/kg - 100 mg/kg range.

Described herein are:
1) A method of inducing apoptosis or cell death in transformed or non-transformed cells in a patient by administering to the cells RNA strands.
2) The method of paragraph 1 wherein the RNA strands are on average between 1kDa and 50kDa in size.
3) The method of paragraph 1 wherein the RNA strands are dsRNA.
4) The method of paragraph 1 wherein the RNA strands are ssRNA.
5) The method of paragraph 3 wherein the dsRNA is pA:pU.
6) The method of paragraph 5 wherein the dsRNA is administered in dosages ranging from the group consisting of 1 - 999 µg/ kg, 1 - 500 µg/kg, 1 - 100 µg/kg, 1-50 µg/kg, 1 - 25 µg/kg, 1- 10 µg/kg and 1 - 5 µg/kg.
7) The method of paragraph 6 wherein the dsRNA is administered intravenously and is administrated in dosages selected from the group consisting of 1 - 999 µg/ kg, 1 - 500 µg/kg, 1 - 100 µg/kg, 1 - 50 µg/kg, 1 - 25 µg/kg, 1 - 10 µg/kg and 1 - 5 µg/kg.
8) The method of paragraph 1 wherein the patient is human and the cells are transformed cells and dsRNA is pA:pU and the dsRNA is injected directly into the cancer cells.
9) The method of paragraph 4 wherein the ssRNA is pA.
10) The method of paragraph 1 wherein the patient is human and the transformed cells are selected from the group consisting of T cell leukemia cells, human monocytic leukemia cells, human adenocarcinoma cells and human lung fibroblasts.
11) A method of inducing cell death or apoptosis in cells by in vivo administration of ssRNA or dsRNA to cells, wherein the ssRNA or dsRNA is smaller on average than 20kDa in size.
12) The method of paragraph 11 wherein the ssRNA or dsRNA is smaller on average than 10kD in size.
13) The method of paragraph 12 wherein the cells are transformed cells.
14) The method of paragraph 12 wherein the ssRNA is pA.
15) The method of paragraph 11 wherein the dsRNA is pA:pU.
16) The method of paragraph 12 wherein the cells are cancer cells.
17) The method of paragraph 1 wherein the RNA strand induces an enhanced cytokine production of TNF - alpha.
18) The method of paragraph 12 wherein the RNA strand induces an enhanced cytokine production of TNF - alpha.
19) The method of paragraph 1 wherein the use of RNA to induce cell death induces an enhanced immune response against the cells.
20) The method of paragraph 1 wherein the RNA is administered *in vivo* to cells and the modes of administration are selected from the group consisting of topically, systemically or by direct injection.
21) A method of inducing apoptosis in cells by administering ssRNA or dsRNA to cells, wherein the ssRNA or dsRNA is smaller than 10KDa in size.
22) The method of paragraph 21 wherein the RNA is ssRNA and is pA.
23) The method of paragraph 21 wherein the RNA is dsRNA and is pA:pU.
24) The method of paragraph 21 wherein the cells are transformed cells.
25) The method of paragraph 21 wherein the induced apoptotic cells induce an enhanced IL-12 cytokine production against similar live cells.
26) The method of paragraph 21 wherein a Th-1 response is directed.
27) The method of paragraph 1 wherein the RNA is dsRNA and is greater than 50 kDa in weight and induces an enhanced IL-12 response.
28) A composition for inducing cell death or apoptosis in transformed cells wherein the composition is comprised of RNA with an average weight between 1 - 50kDa.
29) The composition of paragraph 28 wherein the average weight of the dsRNA or ssRNA is less than 20kDa.
30) The composition of paragraph 28 wherein the average weight of the dsRNA or ssRNA is less than 10kDa.
31) The composition of paragraph 28 wherein the dsRNA is pA:pU.
32) The composition of paragraph 28 wherein the dsRNA induces an enhanced cytokine production of TNF - alpha thereby inducing a T₁ immune response against the transformed cells.
33) A composition for inducing cell death or apoptosis in transformed cells in a patient wherein the composition is comprised of an oligonucleotide wherein the oligonucleotide is comprised of at least two base pairs selected from the group consisting of adenine, uracil, cytosine, guanine and inosine and wherein the oligonucleotide is between 1kDa 50kDa in weight.
34) The composition of paragraph 33 wherein the oligonucleotide is ssRNA.
35) The composition of paragraph 33 wherein the oligonucleotide is dsRNA.
36) The composition of paragraph 33 wherein the ssRNA is pA.
37) The composition of paragraph 35 wherein the oligonucleotide is pA:pU.
38) The composition of paragraph 33 wherein the oligonucleotide is between 1kDa - 10 kDa in weight.
39) The composition of paragraph 33 wherein the oligonucleotide is between 1kDa - 5kDa in weight.
40) Use of a composition in the manufacture of a medicament for inducing cell death or apoptosis in transformed cells wherein the composition is comprised of an oligonucleotide wherein the oligonucleotide is comprised of at least two base pairs selected from the group consisting of adenine, uracil, cytosine, guanine and inosine and wherein the oligonucleotide is between 1kDa - 50kDa in weight.
41) The use of paragraph 40 wherein the oligonucleotide is ssRNA.
42) The use of paragraph 40 wherein the oligonucleotide is dsRNA.
43) The use of paragraph 40 wherein the ssRNA is pA.
44) The use of paragraph 40 wherein the oligonucleotide is pA:pU.
45) The use of paragraph 40 wherein the oligonucleotide is between 1kDa - 10 kDa in weight.
46) The use of paragraph 40 wherein the oligonucleotide is between 1kDa - 5kDa in weight.
47) Use of a composition in the manufacture of a medicament for inducing cell death or apoptosis in transformed cells wherein the composition is comprised of RNA with an average weight between 1 - 50kDa.
48) The use of paragraph 47 wherein the average weight of the dsRNA or ssRNA is less than 20kDa.
49) The use of paragraph 47 wherein the average weight of the dsRNA or ssRNA is less than 10kDa.
50) The use of paragraph 47 wherein the dsRNA is pA:pU.
51) The use of paragraph 47 wherein the dsRNA induces an enhanced cytokine production of TNF - alpha.
52) The use of paragraph 51 wherein the enhanced production of TNF-alpha and induces T₁ immunity against the transformed cells.

### Brief Description of the Drawings

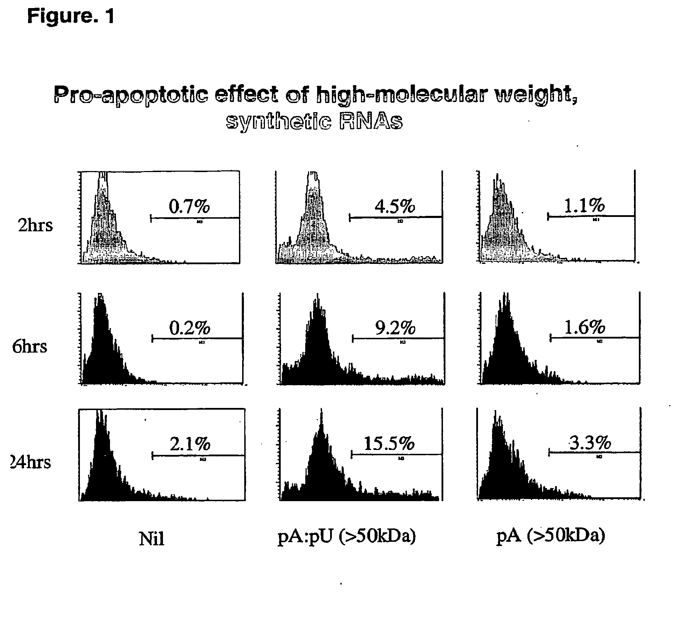

Figure 1 shows the effects of high molecular weight synthetic RNAs on inducing apoptosis in antigen presenting cells (APCs);
Figure 2 shows the effects of low and high molecular weight RNAs on inducing cell death on transformed human monocytic cells;
Figure 3 shows the cytokine profile of TNF - a and IL-12 expression based on human antigen presenting cells;

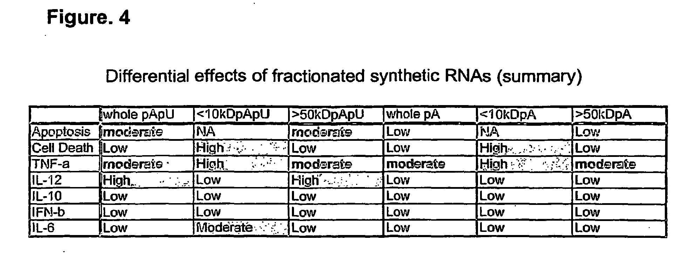

Figure 4 is a table showing the differential effects of fractionated low molecular and high molecular weight RNA;
Figure 5 shows that various concentrations of low molecular weight pA:pU was significantly better at inducing substantial apoptosis and cell death among human T cell leukemia cell lines than the high molecular weight pA:pU, whole pA:pU or the controls;
Figure 6 demonstrates that low molecular weight pA:pU was significantly able to induce apoptosis and cell death among the human T lymphocyte cell lines than either the high molecular weight pA:pU and whole pA:pU and the controls;
Figure 7 demonstrates that low molecular weight pA:pU, particularly at the higher concentration of 100 µg/ml, was able to induce significant levels of cell death and apoptosis among human T cell lymphoblastic leukemia cells;
Figure 8 demonstrates that low molecular weight pA:pU, particularly at concentrations of 100 µg/ml, was significantly better at inducing substantial apoptosis and cell death among human monocytic leukemia cells lines than either the controls or high molecular weight pA:pU;
Figure 9 demonstrates that low molecular weight pA:pU was successful at inducing apoptosis and cell death among human cervical adenocarcinoma cells;
Figure 10 demonstrates that low molecular weight pA:pU was significantly better at inducing substantial apoptosis and cell death among human lung fibroblasts than either the high molecular weight pA:pU, whole pA:pU or the controls;
Figure 11 shows that neither low or high molecular weight pA:pU was able to induce cell death in breast cancer cells;
Figures 12A - 12B shows that neither low nor high molecular weight dsRNA was able to induce cell death or apoptosis in human PBMCs; and,
Figure 13 shows low molecular weight dsRNA fraction and pA:pU of known oligomer length to display cell death inducing effects on monocytic leukemia cells.

### Detailed Description of the Invention:

### Definitions:

As used herein, the following terms and phrases shall have the meanings set forth below:
A "dsRNA" shall mean a double stranded RNA segment which may be comprised of the bases adenine, cytosine, uracil, guanine and inosine and which may be entirely complimentary, partially complementary or a mixed nucleotide strand;
A "ssRNA" shall mean a single stranded RNA segment which may be comprised of the bases adenine, cytosine, uracil, and guanine either uniformly of one nucleotide or of mixed nucleotides;
"Low molecular weight dsRNA or ssRNA" means RNA strands of approximately 10 kDa or less (<10k MW). Preferably, the low molecular weight ssRNA and dsRNA strands of the present invention range between 2 to 40 base pairs;
"High molecular weight dsRNA or ssRNA" means RNA strands of approximately 10 kDa or more and between 10 kDa and 50 kDa but also greater than 50 kDa (>50k MW);
"Whole dsRNA" refers to the dsRNA of any or all molecular weight or oligomer length;
"APC" means antigen presenting cell;
"Cell death" refers to death of a cell from other than apoptosis;
"Apoptosis" refers to programmed cell death;
"Th-1 response" refers to a T - helper 1 response;
"pA:pU" - refers to dsRNA where the RNA strand or segment is comprised of adenine and uracil where the RNA strand or segment is complementary and encompasses RNA strands or segments that are not uniformly complementary;
"-mer" - shorthand term for oligomer;
"HL-1 media" - HL-1 medium (BioWhittaker, Walkersville MD, cat# 344017) with 2 mM L-glutamine (Invitrogen, Carlsbad, CA , cat#11875-085), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023);
"<" means less than; and
">" - means greater than.

### Ex. 1. High Molecular Weight synthetic RNA was able to induce substantial apoptosis of human antigen presenting cells (APC).

Human APCs (THP-1 cells, American Type Culture Collection [ATCC], Manassas, VA) were maintained under conditions as suggested by the ATCC: (a) Media: RPMI 1640 medium with 2 mM L-glutamine (Invitrogen, Carlsbad, CA , cat#11875-085) adjusted to contain10 mM HEPES (Invitrogen, cat# 15630-080), 50 units/ml penicillin-streptomycin (Invitrogen, cat# 15070-063) and 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Coming NY, cat#430641) fresh media substituted every 3 days for up to 4 months when a new culture was started; and (c) temperature: 37 °C, 5% CO₂.

THP-1 cells were suspended in the above described media and differentiated upon addition of 0.05 µM vitamin D3 (CalBiochem, San Diego, CA). Cells were immediately added to sterile 48 well tissue culture plates (BD Falcon, cat# 353078) at a concentration of 0.4 x 10⁶ cells per well (0.5 ml of 0.8 x 10⁶ cells per ml) and allowed to mature for 3 days. At this time, 0.2 mls fresh non-FBS containing HL-1 media replaced the 0.5 mls of media covering the now adherent cells in the microtiter plate wells. The endotoxin tested and fractionated (described separately) high molecular weight (>50kDa) polyadenylic acid-polyuridylic acid (pA:pU, Sigma, cat# P1537, lot#022K4068) or polyadenylic acid (pA, Sigma, cat# P9403, lot#022K4022) was added to cells in media at 50µg/ml. Cells were allowed to incubate in sterile conditions at 37°C / 5% CO₂ for 2, 6 or 24 hrs. At these same time points, supernatants were collected and frozen (-70°C) for further analysis at a later time. Sterile, cold phosphate buffered saline (PBS, Sigma, cat# D8537) was added gently over the cells followed by staining with Yo-Pro (Molecular Probes, Eugene, OR, cat#Y-3603, 0.15µM final concentration) an indicator of apoptosis. After 10 minutes of incubation, cells were washed once and suspended in buffer containing 2% FBS and stored on ice. Cells were then analyzed for apoptosis with a FACS Calibur flow cytometer (BD Bioscience, San Jose, CA) using appropriate filters to analyze cellular uptake of Yo-Pro in comparison to appropriate control cells.

The results demonstrate that higher molecular weight dsRNA fractions, pA:pU (>50kDa), were able to induce substantial apoptosis of human antigen presenting cells. In the control at time intervals of 2, 6 and 24 hours, only minimal apoptosis was observed. However, a significantly higher level of apoptosis was observed for pA (>50kDa) at intervals of 2, 6 and 24 hours and a dramatically higher induction of apoptosis was observed in cells incubated with pA:pU (>50kDa).

### Ex. 2. Low dsRNA molecular weight fractions display apoptosis and cell death inducing effects on transformed human monocytic cells.

Human APCs (THP-1 cells, American Type Culture Collection [ATCC], Manassas, VA) were maintained under conditions as suggested by the ATCC: Media: RPMI 1640 medium with 2 mM L-glutamine (Invitrogen, Carlsbad, CA, cat# 11875-085) adjusted to contain 10 mM HEPES (Invitrogen, cat# 15630-080), 50 units / ml penicillin-streptomycin (Invitrogen, cat# 15070-063) and 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); temperature: 37°C, 5% CO₂. Continuous culture: in cell culture flasks (Coming, Corning, NY; cat#430641) fresh media substituted every 3 days for up to 4 months when a new culture was started.

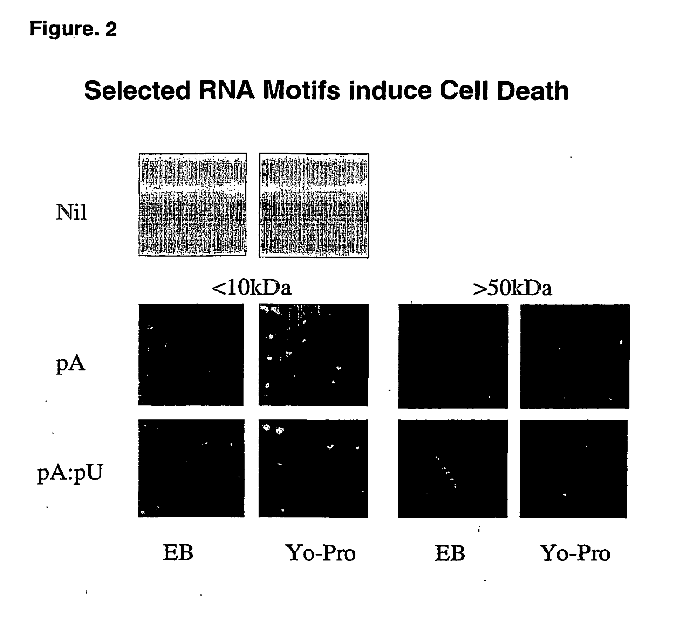

THP-1 cells were suspended and differentiated in above media to which 0.05µM vitamin D3 (CalBiochem, San Diego, CA) was added. Cells were immediately added to sterile 48 well tissue culture plates (BD Falcon, cat# 353078) at a concentration of 0.4 x 10⁶ cells per well and allowed to mature for 3 days. At this time, 0.2 mls fresh non-FBS containing HL-1 media replaced the 0.5 mls of media covering the now adherent cells in the microtiter plate wells. The endotoxin tested and fractionated (described separately) high molecular weight (>50kDa) polyadenylic acid-polyuridylic acid (pA:pU, Sigma, cat# P1537, lot#022K4068) or polyadenylic acid (pA, Sigma, cat# P9403, lot#022K4022) was added to cells in media at 50mg/ml. Cells were allowed to incubate in sterile conditions at 37°C / 5% CO₂ for 2, 6 or 24hrs. At these same time points, supernatants were collected and frozen (-70°C) for further analysis at a later time. Sterile, cold phosphate buffered saline (PBS, Sigma, cat# D8537) was added gently over the cells followed by staining with ethidium bromide (Sigma, cat# 46067, 1.5µg/ml final concentration) an indicator of cell death and/or Yo-Pro (Molecular Probes, Eugene, OR, cat#Y-3603, 0.15µM final concentration) an indicator of apoptosis. After 10 minutes of incubation, cells were analyzed by fluorescent microscopy using appropriate filters to differentiate between apoptotic and dead cells from live cells and images taken (as shown in Figure 2) using a fluorescent microscope with appropriate filters and image analysis , system using Image Pro software (Media Cybernetics, Carlsbad, CA).

The results demonstrate that low molecular weight RNA strands (pA and pA:pU < 10kDa) induce cell death significantly more effectively than higher molecular weight RNA strands (pA and pA:pU > 50kDa) and both induced cell death more extensively than the control (nil). The results also indicate that low molecular weight ssRNA and dsRNA (<10kDa) are more successful than high molecular weight ssRNA and dsRNA (>50kDa) at inducing apoptosis in cells and are dramatically more successful in inducing apoptosis than the control.

It is theorized that low molecular weight RNAs (<10kDa) enter into the cell without a receptor while high molecular weight RNAs (>50kDa) enter into a cell by engaging a cellular receptor. It is also theorized that the uptake by cellular receptors of high molecular weight RNAs depend on the secondary structure of the RNA strand.

### Example 3. Cytokine profile of human APC following exposure to RNA motif fractions.

Human APCs (THP-1 cells, American Type Culture Collection [ATCC], Manassas, VA) were maintained under conditions as suggested by the ATCC: Media: RPMI 1640 medium with 2 mM L-glutamine (Invitrogen, Carlsbad, CA , cat#11875-085) adjusted to contain 10 mM HEPES (Invitrogen, cat# 15630-080), 50 units / ml penicillin-streptomycin (Invitrogen, cat# 15070-063) and 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019) Temperature: 37 °C, 5% CO₂. Continuous culture: in cell culture flasks (Coming, Coming, NY, cat#430641) fresh media substituted every 3 days for up to 4 months when a new culture was started.

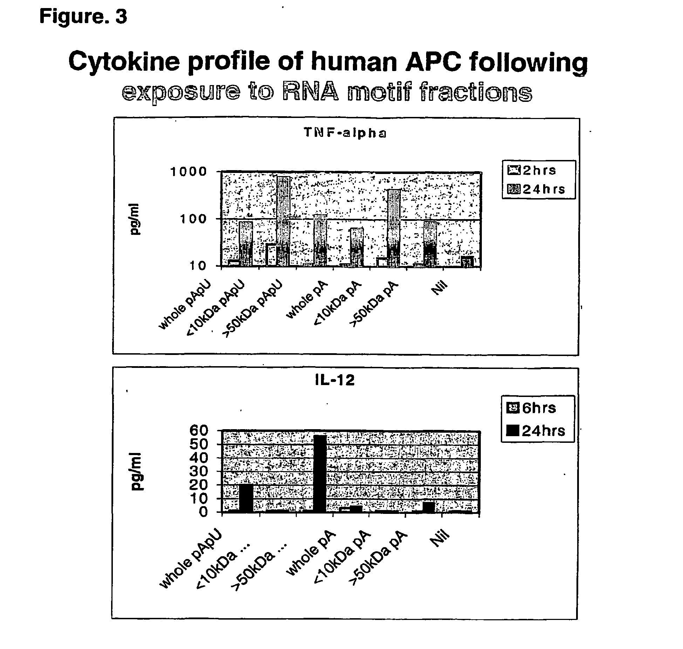

THP-1 cells were suspended and differentiated in above media to which 0.05µM vitamin D3 (CalBiochem, San Diego, CA) was added. Cells were immediately added to sterile 48 well tissue culture plates (BD Falcon, cat# 353078) at a concentration of 0.4 x 10⁶ cells per well and allowed to mature for 3 days. At this time, 0.2 mls fresh non-FBS containing HL-1 media replaced the 0.5 mls of media covering the now adherent cells in the microtiter plate wells. The endotoxin tested whole and fractionated (described separately) high molecular weight (>50kDa) polyadenylic acid-polyuridylic acid (pA:pU, Sigma, cat# P1537, lot#022K4068) or polyadenylic acid (pA, Sigma, cat# P9403, lot#022K4022) was added to cells in media at 50mg/ml. Cells were allowed to incubate in sterile conditions at 37°C / 5% CO₂ for 2, 6 or 24hrs. At these same time points, supernatants were collected and frozen (-70°C) prior to cytokine analysis. ELISA assays for human IL-12 (Biosourse International, Camarillo, CA, cat # KHC0121) or human TNF-alpha (Biosourse International, cat# KHC3011) were used to determine cytokine concentrations in cell supernatants (Figure 3). ELISA assays were carried out per instructions provided by manufacturer.

The results demonstrate (see Figure 3) that low molecular weight ssRNA and dsRNA (<10kDa pA:pU and pA) motifs are able to induce the pro-inflammatory cytokine TNF-alpha. Of low molecular weight RNA motifs, dsRNAs (<10kDa pA:pU) were somewhat more successful in inducing TNF-alpha than ssRNAs (<10kDa pA). Higher molecular weight RNA motifs (>50kDa pA:pU and > 50kDa pA) were also able to significantly induce TNF-alpha significantly better than the control. Thus not only do low and higher molecular weight RNAs induce cell death and apoptosis but they are able to induce pro-inflammatory cytokine TNF-alpha thereby facilitating an immune response against cells thereby resulting in both a cytotoxic effect against the cells followed by directing a subsequent or enhanced immune response against the cells. In contrast to the pro-inflammatory cytokine TNF-alpha production following treatment with low molecular weight single or double stranded RNA, the results demonstrate that higher molecular weight (> 50kDa pA:pU) was able to induce a significant IL-12 production, when compared to any other treatment or control (nil), which is indicative of Th1 response modulation.

### Example 4. Demonstrates how synthetic RNA is fractionated, purified and the concentration ascertained prior to use.

The bulk synthetic RNA material is obtained by standard methods of organic synthesis and can be obtained commercially. For example, RNA motif polyadenylic acid - polyuridylic acid (pA:pU, Sigma, cat#P1537, lot #22K4068) or polyadenylic acid (pA, Sigma, cat# p9403, lot#22K4022) can be obtained from Sigma. The fractions used in , many of these Examples comprise synthetic RNA of less than 20 bp to approximately 100 bp in size.

The synthetic RNA material is fractionated by a series of centrifugation steps through filters of defined porosity. Per the manufacturer's instructions, approximately 20 mgs of the pA:pU was placed into a Centriprep YM-50, 50K MWCO (Amicon, cat #4323) for centrifugal filtration and centrifuged at 1500 Xg for 15 minutes. The filtrate, of less than 50K MW, was collected and the YM-50 was spun two additional times, as above, with both the filtrate (<50K MW) and retentate (>50K MW) saved. The filtrate (<50K MW) was then placed into a Centriprep YM-10, 10K MWCO (Amicon, cat #4304) and centrifuged at 3000 Xg, two times, for 20 minutes and both the filtrate (<10K MW) and retentate (>10K MW) was saved.

After fractionating the bulk synthetic RNA material, the material is dissolved in sterile endotoxin-free saline (e.g., Dulbecco's Phosphate Buffered Saline [PBS], sterile, endotoxin tested, [Sigma, cat#D8537]) and passed through separate endotoxin removal columns such as AffinitiPak Detoxi-Gel Endotoxin Removing Gel Column (Pierce Chemical, cat # 20344) and effluent was collected. The effluent is passed through the endotoxin removal columns until endotoxin levels were below 10 EU/mg as measured by the concentration of lipopolysaccharide (LPS). The measurement of LPS is carried out by Limulus assay (e.g., Limulus Amebocyte Lysate ["LAL"] chromogenic assay [BioWhitakker, Kinetic-QCL, cat #50 - 650 U]).

Following endotoxin removal, the motifs were diluted 1:100 in PBS and absorbance at 260 nm with a Beckman DU-640 Spectrophotometer was obtained on a known concentration of motif in PBS (Dulbecco's Phosphate Buffered Saline, Sigma, cat # D8537). This yielded the following extinction coefficients ("ec"):
a) pA:pU, (Sigma, Lot #22K4068): ec=0.0601;
b) pA, (Sigma, Lot#22K4022): ec= 0.0575.
Bioactivity of the motifs was then determined as shown in the Examples.

### A. Leukemia Cell Lines

### Example 5 - Low Molecular Weight dsRNA Fractions Display Apoptosis and Cell Death Inducing Effects on T Cell Leukemia Cell Lines.

Human acute T cell leukemia cell lines (Jurkat) were obtained from American Type Culture Collection (ATCC, Manassas, VA) and maintained under conditions as suggested by the ATCC: (a) Media: RPMI 1640 medium with 2 mM L-glutamine (Invitrogen, Carlsbad, CA, cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Corning NY, cat#430641) fresh media substituted every 3 days for up to 4 months when a new culture was started; and (c) temperature: 37 °C, 5% CO₂. Jurkat cells are a nontransfected human acute T cell leukemia cell line which mimic human T cell leukemias in vivo. Selective killing of this and other leukemias would be of clinical benefit.

The T cell leukemia lines were then placed in 48 well plates at a concentration of 0.4 x 10⁶ cells per well, approximately 80% confluence, in FBS containing media at and allowed to mature for 3 - 4 days. The following day, the media was changed to 0.2 mls fresh non-FBS containing HL-1 media The cells were then pulsed with the endotoxin tested and fractionated dsRNA (pA:pU) (as taught in Example 4) at concentrations ranging from 10, 30 to 100 µg/ml for 6 hours followed by the media being replaced with fresh HL-1 media and incubated overnight in sterile conditions at 37° C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and suspended in buffer containing 2% FBS and stored on ice. Cells were then analyzed for apoptosis and cell death with a FACS Calibur flow cytometer (BD Bioscience, San Jose, CA) using appropriate filters to analyze cellular uptake of Yo-Pro and ethidium bromide in comparison to appropriate control cells.

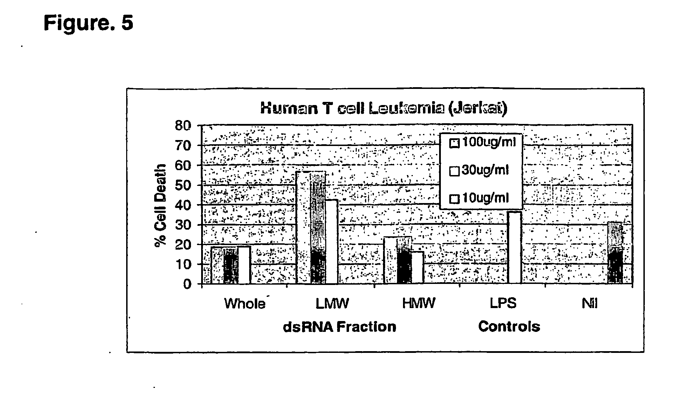

The results, as shown in Figure 5, demonstrate that low molecular weight pA:pU, which is indicated as mixtures under pA:pU of 10kD or approximately 40 basepairs or less, was significantly better at inducing substantial apoptosis and cell death among human T cell leukemia cell lines than the high molecular weight pA:pU, whole pA:pU containing oligonucleotides over a wide range of base pair lengths (from a few through a few thousand) and the controls. In particular, the higher concentrations of LMW, 30 µg/ml and 100 µg/ml, were more successful at inducing cell death and apoptosis than the lower concentration LMW pA:pU (10 µg/ml).

Example 6 - Low Molecular Weight dsRNA fractions display apoptotic and cell death inducing effects on human T lymphocytes.

Human T lymphocyte cell lines (ATL-2) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA, cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Corning, Coming NY, cat#430641) fresh media substituted every 3 days for up to 4 months when a new culture was started; and (c) temperature: 37 °C, 5% CO₂. ATL-2 cells are a nontransfected human T cell leukemia cell line which mimics human T cell leukemias in vivo. Selective killing of these and other leukemias would be of significant clinical benefit. The T lymphocyte cell lines were then placed in 48 well plates at a concentration of approximately 0.4 x 10⁶ cells per well in FBS containing media at approximately 80% confluence and allowed to mature for 3 - 4 days. The following day, the media was changed to non-FCS containing HL-1 media of approximately 0.2 mls per well as outlined above but without FBS.

The cells were then pulsed with the purified and fractionated dsRNA (pA:pU) (see Example 4) at concentrations of 10, 30 and 100 µg/ml for 6 hours followed by the media being replaced with fresh HL-1 media and incubated overnight in sterile conditions at 37° C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analyzed for apoptosis with a FACS Calibur flow cytometer (BD Bioscience. San Jose, CA) using appropriate filters to analyze cellular uptake of Yo-Pro and ethidium bromide in comparison to appropriate control cells.

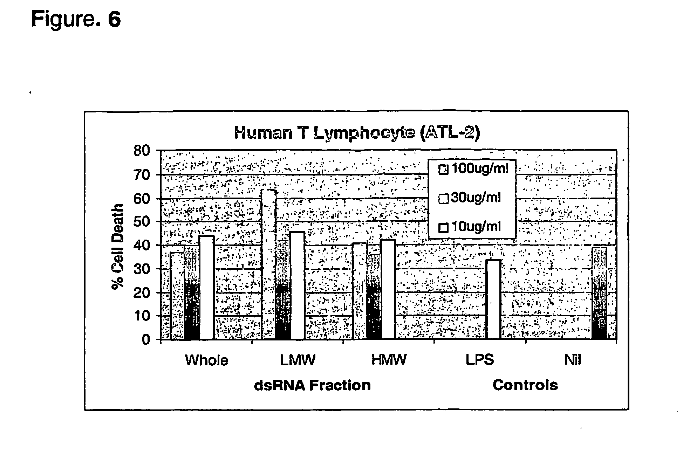

The results, as shown in Figure 6, demonstrate that low molecular weight pA:pU, which is indicated as mixtures under pA:pU of 10kD or less, was significantly able to induce apoptosis and cell death among the human T lymphocyte cell lines than either the high molecular weight pA:pU and whole pA:pU and the controls. The higher concentration of 100 µg/ml of LMW pA:pU was significantly more successful in inducing cell death and apoptosis in T lymphocyte cell lines than lower concentrations of pA:pU.

### Ex. 7 - Low Molecular Weight dsRNA fractions demonstrate apoptotic and cell death inducing effects on human T cell lymphoblasts.

Human T cell lymphoblastic leukemia cells (CEM) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA , cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Corning, Corning NY, cat#430641) fresh media substituted every 3 days for up to 4 months when a new culture was started; and (c) temperature: 37 °C, 5% CO₂. CEM cells are a nontransfected human T cell lymphoblastic leukemia cell line which mimics human T cell leukemias in vivo. Selective killing of this and other leukemias would be of clinical benefit.

The human T cell lymphoblastic leukemia cells were then placed in 48 well plates at a concentration of 0.4 x 10⁶ cells per well, approximately 80% confluence, in FBS containing media and allowed to mature for 3 - 4 days. The following day, the media was changed to non-FCS containing HL-1 media, 0.2 mls per well. The cells were then pulsed with endotoxin tested and fractionated dsRNA (pA:pU) (see Example 4) at concentrations of 10 µg/ml, 30 µg/ml and 100 µg/ml for 6 hours followed by the media being replaced with fresh HL-1 media and incubated overnight in sterile conditions at 37° C/5% CO₂. Cells were then resuspended in cold, phosphate buffer saline (PBS, Sigma, cat# D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analysed for apoptosis and cell death with a FACS Calibur flow cytometer (BD Bioscience, San Jose, CA) using appropriate filters to analyze cellular uptake of Yo-Pro and ethidium bromide in comparison to appropriate control cells.

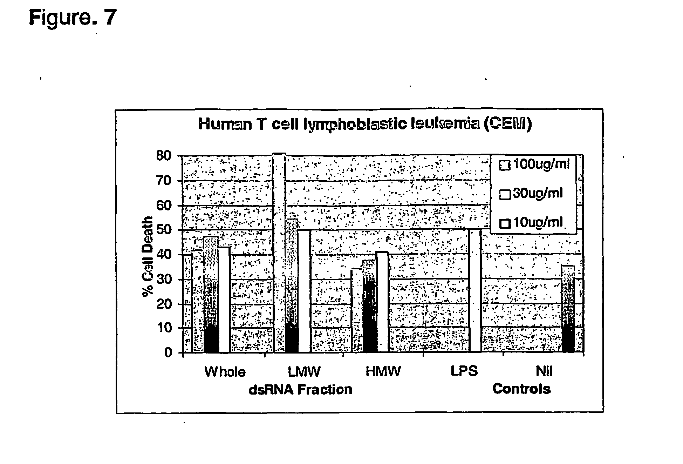

The results, as shown in Figure 7, demonstrate that low molecular weight pA:pU, particularly at the higher concentration of 100 µg/ml, was able to induce significant levels of cell death and apoptosis among human T cell lymphoblastic leukemia cells lines than the controls or the high molecular weight pA:pU and whole pA:pU.

### Example 8 - Low molecular weight dsRNA fractions demonstrate apoptotic and cell death inducing effects on human monocytic leukemia cells.

Human monocytic leukemia cells (THP-1) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA , cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Corning, Corning NY, cat#430641) fresh media substituted every 3 days for up to 4 months when a new culture was started; and (c) temperature: 37°C, 5% CO₂. THP-1 cells are a nontransfected human monocytic leukemia cell line which mimics human monocytic leukemias *in vivo.* Selective killing of this and other leukemias would be of clinical benefit

The human monocytic leukemia cells were then placed in 48 well plates at a concentration of 0.4 x 10⁶ cells per well, approximately 80% confluence in FBS containing media and allowed to mature for 3 - 4 days. The following day, the media was changed to non-FBS containing HL-1 media, 0.2 mls per well.

The cells were then pulsed with the endotoxin tested and fractionated dsRNA (pA:pU) (see Example 4) at concentrations of 10, 30 and 100 µg/ml for 6 hours followed by the media being replaced with fresh HL-1 media and incubated overnight in sterile conditions at 37° C/5% CO₂ Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analyzed for apoptosis with a FACS Calibur flow cytometer (BD Bioscience. San Jose, CA) using appropriate filters to analyze cellular uptake of Yo-Pro and ethidium bromide in comparison to appropriate control cells.

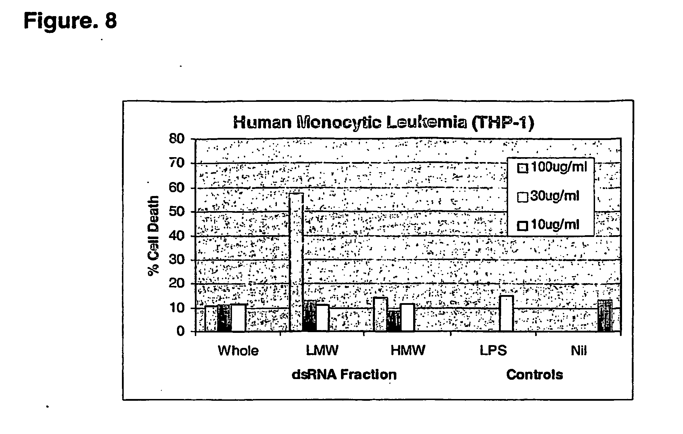

The results, as shown in Figure 8, demonstrate that low molecular weight pA:pU, particularly at concentrations of 100 µg/ml, was significantly better at inducing substantial apoptosis and cell death among human monocytic leukemia cells lines than either the controls or high molecular weight pA:pU and whole pA:pU.

### B. Non-Leukemia Cell Lines

### Example 9 - Low and high molecular weight dsRNA fractions were unsuccessful at inducing either cell death or apoptosis in human adenocarcinoma cell lines at a rate greater than the control.

Human cervical adenocarcinoma cell lines (HeLa) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA , cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Coming NY, cat#430641) fresh media substituted every 3 days for up to 4 months when a new culture was started; and (c) temperature: 37 °C, 5% CO₂. HeLa cells are a nontransfected cervical adenocarcinoma human cell line which mimics adenocarcinoma in vivo. Selective killing of this and other carcinomas would be of clinical benefit.

The human cervical adenocarcinoma cell lines were then placed in 48 well plates at a concentration of 0.4 x 10⁶ cells per well, approximately 80% confluence, in FBS containing media and allowed to mature for 3 - 4 days. The following day, the media was changed to non-FBS containing HL-1 media, 0.2 mls per well. The cells were then pulsed with the endotoxin tested and fractionated dsRNA (pA:pU) (see Ex. 4) at concentrations ranging from 10 - 100 µg/ml for 6 hours followed by the media being replaced with fresh HL-1 media and incubated overnight in sterile conditions at 37° C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analyzed for apoptosis and cell death with a FACS Calibur flow cytometer (BD Bioscience, San Jose, CA) using appropriate filters to analyze cellular uptake of Yo-Pro and ethidium bromide in comparison to appropriate control cells.

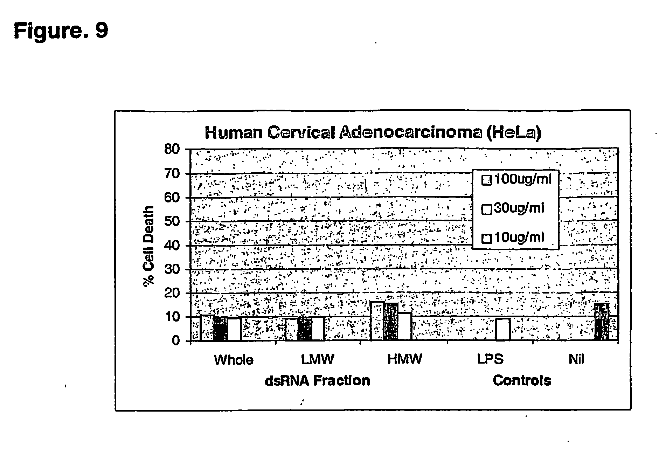

The results, as shown in Figure 9, demonstrate that low molecular weight pA:pU was no more successful at inducing apoptosis and cell death among human cervical adenocarcinoma cells than either the high molecular weight pA:pU or controls.

### Example 10 - Low molecular weight pA:pU, at a concentration of 100 µg/ml, was significantly better at inducing substantial apoptosis and cell death among human lung fibroblasts than either the high molecular weight pA:pU and whole pA:pU and the controls.

Human Lung Fibroblasts ("WI 38") were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA , cat#11875-085) adjusted to contain 10 mM HEPES (Invitrogen, cat# 15630-080), 50 units/ml penicillin-streptomycin (Invitrogen, cat# 15070-063) and 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Coming NY, cat#430641) fresh media substituted every 2-3 days for up to 50 days when a new culture was started; and (c) temperature: 37 °C, 5% CO₂. WI-38 is a human lung fibroblast cell line which mimics a lung carcinoma.

The human lung fibroblasts were then placed in 48 well plates at a concentration of 0.4 x 10⁶ cells per well, approximately 80% confluence, in FBS containing media and allowed to mature for 3 - 4 days. The following day, the media was changed to non-FCS containing HL-1 media, 0.2 mls per well. The cells were then pulsed with the endotoxin tested and fractionated dsRNA (pA:pU) (see Example 4) at concentrations ranging from 10 - 100 µg/ml for 6 hours followed by the media being replaced with fresh HL-1 media and incubated overnight in sterile conditions at 37° C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analyzed for apoptosis and cell death with a FACS Calibur flow cytometer (BD Bioscience. San Jose, CA) using appropriate filters to analyze cellular uptake of Yo-Pro and ethidium bromide in comparison to appropriate control cells.

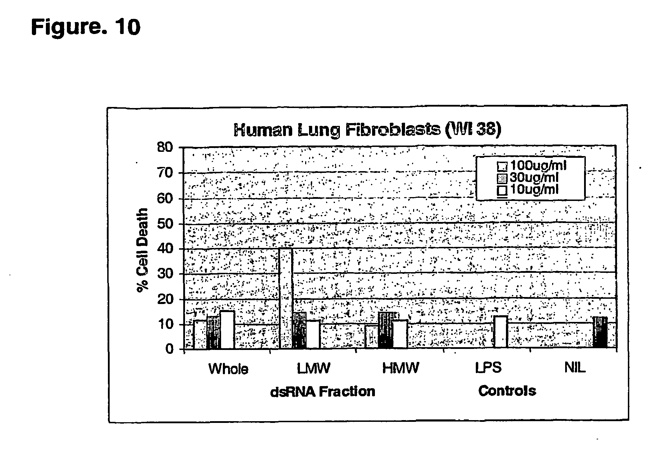

The results, as shown in Figure 10, demonstrate that low molecular weight pA:pU, at a concentration of 100 µg/ml, was significantly better at inducing substantial apoptosis and cell death among human lung fibroblasts than either the high molecular weight pA:pU and whole pA:pU and the controls.

### Example 11 - Neither high nor low molecular weight dsRNA or ssRNA was more successful at inducing cell death or apoptosis in human breast cancer cells than the control.

Human breast cancer cell lines (SKBR - 3) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA , cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Coming NY, cat#430641) fresh media substituted every 3 days for up to 4 months when a new culture was started; and (c) temperature: 37 °C, 5% CO₂.

The human breast cancer cell lines were then placed in 48 well plates at a concentration of 0.4 x 10⁶ cells per well, approximately 80% confluence, in FBS containing media and allowed to mature for 3 - 4 days. The following day, the media was changed to non-FCS containing HL-1 media, 0.2 mls per well. The cells were then pulsed with the endotoxin tested and fractionated dsRNA (pA:pU) (see Example 4) at concentrations ranging from 10 - 100 µg/ml for 6 hours followed by the media being replaced with fresh HL-1 media and incubated overnight in sterile conditions at 37°C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration)an indicator of cell death ). After 10 minutes of incubation, cells were washed once and resuspended in buffer containing 2% FBS and stored on ice. Cells were then analyzed for apoptosis and cell death with a FACS Calibur flow cytometer (BD Bioscience. San Jose, CA) using appropriate filters to analyze cellular uptake of Yo-Pro and ethidium bromide in comparison to appropriate control cells.

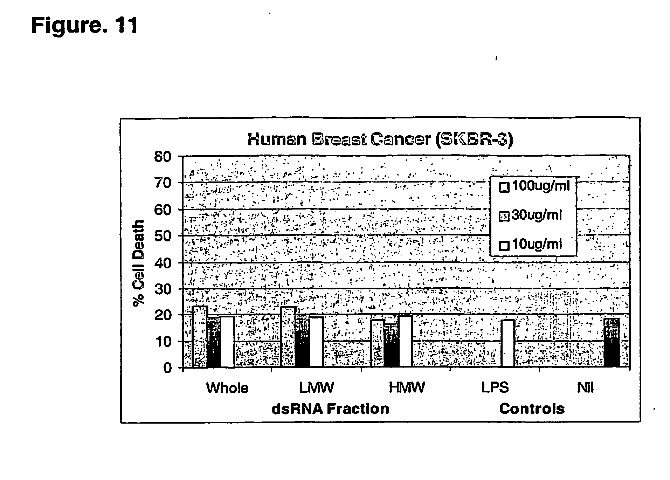

The results, as shown in Figure 11, demonstrate that neither low molecular weight pA:pU, high molecular weight pA:pU or whole pA:pU was able to induce cell death in breast cancer cells any more successfully than the controls.

### Ex. 12 - Low or high molecular weight dsRNA was unable to induce cell death or apoptosis in human PBMCs any greater than either high molecular weight dsRNA or the control.

The Histopaque density gradient medium (Sigma, cat#1077-1) was brought to room temperature and inverted several times to ensure thorough mixing. 3 mL of Histopaque was added to a 15ml centrifuge tube. 5-6 mL of a human blood sample obtained from a human donor (Donors 1 and 2) was carefully layered on the Histopaque and then centrifuged at 400 x g for 30min at 20°C. Using a sterile pipette, the white lymphocyte layer was removed with as little Histopaque and plasma contamination as possible.

The lymphocyte layer was then transferred to a new 15 mL centrifuge tube and 3 volumes of PBS was added to the lymphocytes. The lymphocytes were then resuspended and the tube centrifuged at 1500 RPM for 10 min at 20°C. The supernatant was then decanted and the lymphocytes gently resuspended using a pipette. The tube was then centrifuged again at 1200 RPM for 10 min at 20°C. The supernatant was decanted and the lymphocytes were gently resuspended in HL-1 media. Once again, the tube was centrifuged at 1000 RPM for 10 min at 20°C and resuspend in HL-1 media.

The lymphocytes were then counted and plated in a 48 well plate at 4X10⁵/well in HL-1 media. The fractionated and purified RNA motifs (pA:pU) (see Example 4) were added at 10, 30 and 100 µg/ml final concentration or LPS (E.coli 055:B5) at 100ng/ml to the cells and allowed to incubate at 37°C, 5% CO₂ overnight.

The cells were then resuspended and stained with ethidium bromide and/or Yo-Pro as follows: 100µl of sterile, cold phosphate buffered saline (PBS, Sigma, cat# D8537) was added to the cells followed by staining with ethidium bromide (Sigma, cat# 46067, 1.5µg/ml final concentration) an indicator of cell death and/or Yo-Pro (Molecular Probes, Eugene OR, cat#Y-3603, 0.15µM final concentration) an indicator of apoptosis. The cells were incubated for 10 minutes on ice followed by resuspension in PBS with 2% FBS with subsequent analysis for apoptosis / cell death with a FACS Calibur flow cytometer (BD Bioscience, San Jose CA) using appropriate filters to analyze cellular uptake of Yo-Pro and ethidium bromide in comparison to appropriate controls (LPS, 100ng/ml or Nil, HL-1 media alone.) Cells were also analyzed by fluorescent microscopy using appropriate filters to differentiate between apoptotic and dead cells and images taken using an image analysis system running Image Pro software (Media Cybernetics, Carlsbad CA).

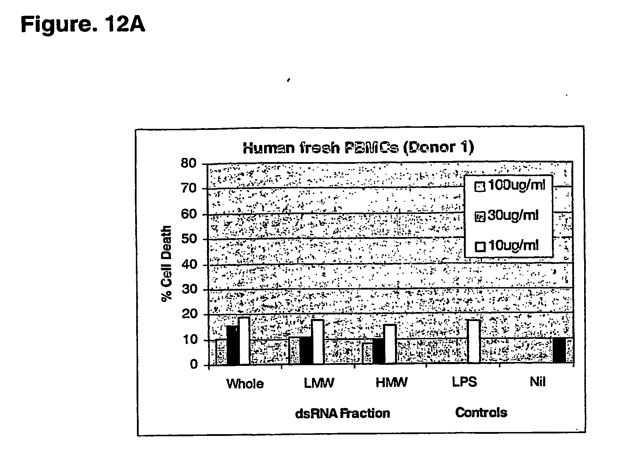

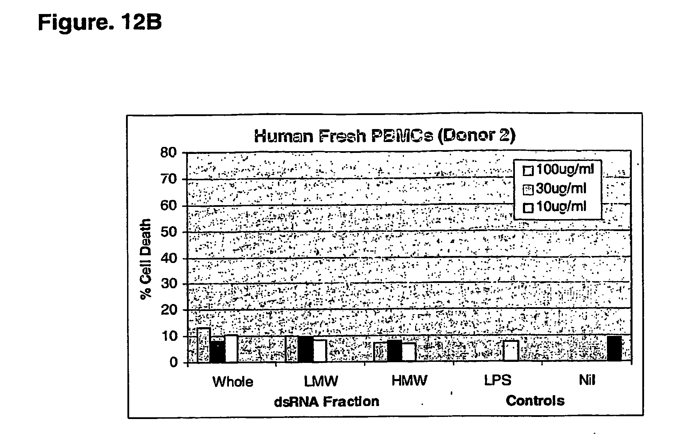

The results in Figures 12A - 12B show that low or high molecular weight dsRNA was not any more capable of inducing cell death or apoptosis in human PBMCs than either high molecular weight dsRNA or the control.

### Example 13 - Low molecular weight dsRNA fraction and dsRNA of known oligomer length display cell death inducing effects on monocytic leukemia cells.

Human monocytic leukemia cells (THP-1) were obtained from American Type Culture Collection [ATCC], Manassas, VA) and maintained under conditions as suggested by the ATCC: (a) Media: RPMI 1640 medium with 2 mM L-glutamine (Invitrogen, Carlsbad, CA, cat#11875-085) adjusted to contain 10 mM HEPES (Invitrogen, cat# 15630-080), 50 units/ml penicillin-streptomycin (Invitrogen, cat# 15070-063), 1.0 mM sodium pyruvate (Invitrogen, cat# 11360-070) and supplemented with 0.05 mM 2-mercaptoethanol (Invitrogen, cat# 21985-023), fetal bovine serum (FBS) 10% (Invitrogen, cat# 26170-019); (b) continuous culture: in cell culture flasks (Coming, Coming NY, cat#430641) fresh media substituted every 3 days for up to 4 months when a new culture was started; and (c) temperature: 37 °C, 5% CO₂. THP-1 cells are a nontransfected human monocytic leukemia cell line that mimics human monocytic leukemias in vivo. Selective killing of this and other leukemias would be of significant clinical benefit

The human monocytic leukemia cells were then placed in 48 well plates at a concentration of 0.4 x 10⁶ cells per well, approximately 80% confluence in FBS containing media and allowed to mature for 3 - 4 days. The following day, the media was changed to non-FBS containing HL-1 media, 0.2 mls per well.

The cells were then pulsed with the endotoxin tested whole and fractionated low molecular weight (<10k MW) dsRNA (pA:pU) (see Example 4) at a concentration of 100 µg/ml, with adenylic acid (Sigma, cat #A-1752), or uridylic acid (Sigma, cat# U-1752) at 50, 100, 200 and 400 µg/ml in sterile PBS, or polyA:polyU of 5-mer, 10-mer or 20-mer annealed nucleotide (oligomer) lengths (Ambion, Austin TX) at 50, 100, 200 and 400 µg/ml in sterile PBS. In addition LPS (200ng/ml) or HL-1 media alone were used as controls. Samples were incubated overnight in sterile conditions at 37° C/5% CO₂. Cells were then resuspended in cold, phosphate buffer saline (PBS, Sigma, cat#D8537) which was added gently over the cells followed by staining with Yo-Pro (Molecular Probes, Eugene, OR, cat #Y-3603, 0.15 µM final concentration) an indicator of apoptosis and ethidium bromide (Sigma, cat# 46067, 1.5µg/ml final concentration) an indicator of cell death. After 10 minutes of incubation, cells were washed once and resuspended in buffer containing 2% FBS and stored on ice. Cells were then analyzed for apoptosis with a FACS Calibur flow cytometer (BD Bioscience. San Jose, CA) using appropriate filters to analyze cellular uptake of Yo-Pro and ethidium bromide in comparison to appropriate control cells.

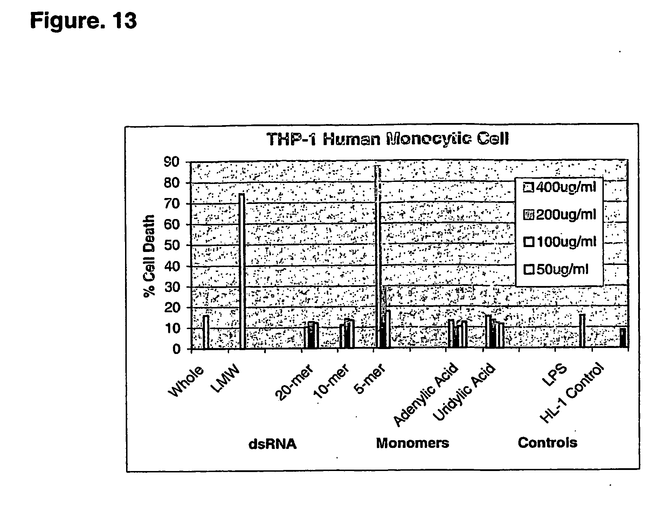

The results, as shown in Figure 13, demonstrate that while low molecular weight (<10k) pA:pU at a concentration of 100 µg/ml was able to induce substantial necrotic cell death as previously shown, the pA:pU 5-mer at concentrations of 200µg or 400µg was able to induce necrotic cell death in slightly higher amounts and in sharp contrast to the cell death induced by the 10-mer or 20-mer pA:pU, the adenylic acid or uridylic acid or controls. This also demonstrates that, as the monomeric adenylic acid or uridylic acid showed only background necrotic activity, the initial pA:pU oligomeric format is important. In addition the low molecular weight (<10k MW) pA:pU fraction may have oligomers of less than 10-mer length from which it's necrotic activity is due.

## Claims

1. RNA for use in a method of anti-leukemia therapy, the method involving inducing non-apoptotic cell death in leukemia cells, wherein the RNA is pA:pU having a molecular weight of 10kDa or less, or wherein the RNA is pA having a molecular weight of 10kDa or less.

2. The RNA for use in a method of anti-leukemia therapy according to claim 1, wherein the RNA induces enhanced cytokine production of TNF-alpha.

3. The RNA for use in a method of anti-leukemia therapy according to claim 1 wherein the RNA has a molecular weight of between 1kDa and 10kDa.

4. The RNA for use in a method of anti-leukemia therapy according to claim 3 wherein the RNA has a molecular weight of between 1kDa and 5kDa.

5. The RNA for use in a method of anti-leukemia therapy according to claim 1 wherein the RNA is a pA:pU 5-mer.

6. The RNA for use in a method of anti-leukemia therapy according to claim 1 wherein the RNA is pA:pU having oligomers of less than 10-mer length.

7. The RNA for use in a method of anti-leukemia therapy according to any one of the preceding claims wherein the RNA is pA:pU.

8. The RNA for use in a method of anti-leukemia therapy according to any one of claims 1-4 wherein the RNA is pA.

9. The RNA for use in a method of anti-leukemia therapy according to any one of claims 1-8 wherein the leukemia cells are T cell leukemia cells or human monocytic leukemia cells.

10. The RNA for use in a method of anti-leukemia therapy according to any one of claims 1 to 9 wherein the RNA is to be administered by topical administration, systemic administration or by direct injection into a tumor.

11. The RNA for use in a method of anti-leukemia therapy according to claim 10 wherein the composition comprises a dosage of pA or pA:pU in the range 1-999µg/kg.

12. The RNA for use in a method of anti-leukemia therapy according to claim 11 wherein the composition comprises a dosage of pA or pA:pU in the range 1-500µg/kg.

13. The RNA for use in a method of anti-leukemia therapy according to any one of the preceding claims wherein the patient is a human.

14. Use of RNA in the manufacture of a composition for use in a method of anti-leukemia therapy, the method involving inducing non-apoptotic cell death in leukemia cells in a patient, wherein the RNA is pA:pU having a molecular weight of 10kDa or less, or wherein the RNA is pA having a molecular weight of 10kDa or less.

15. The use of claim 14 wherein the RNA induces enhanced cytokine production of TNF-alpha.

16. The use of claim 14 wherein the RNA has a molecular weight of between 1kDa and 10kDa.

17. The use of claim 16 wherein the RNA has a molecular weight of between 1kDa and 5kDa.

18. The use of claim 14 wherein the RNA is a pA:pU 5-mer.

19. The use of claim 14 wherein the RNA is pA:pU having oligomers of less than 10-mer length.

20. The use of any one of claims 14 to 19 wherein the RNA is pA:pU.

21. The use of any one of claims 14 to 17 wherein the RNA is pA.

22. The use of any one of claims 14 to 21 wherein the leukemia cells are T cell leukemia cells or human monocytic leukemia cells.

23. The use of any one of the preceding claims wherein the patient is a human.

## Patentansprüche

1. RNA zur Verwendung in einem Verfahren der Anti-Leukämie-Therapie, wobei das Verfahren das Induzieren von nicht apoptotischem Zelltod bei Leukämiezellen umfasst, worin die RNA pA:pU mit einem Molekulargewicht von 10 kDa oder weniger ist oder worin die RNA pA mit einem Molekulargewicht von 10 kDa oder weniger ist.

2. RNA zur Verwendung in einem Verfahren der Anti-Leukämie-Therapie nach Anspruch 1, worin die RNA erhöhte Zytokinproduktion von TNFα induziert.

3. RNA zur Verwendung in einem Verfahren der Anti-Leukämie-Therapie nach Anspruch 1, worin die RNA ein Molekulargewicht zwischen 1 kDa und 10 kDa aufweist.

4. RNA zur Verwendung in einem Verfahren der Anti-Leukämie-Therapie nach Anspruch 3, worin die RNA ein Molekulargewicht zwischen 1 kDa und 5 kDa aufweist.

5. RNA zur Verwendung in einem Verfahren der Anti-Leukämie-Therapie nach Anspruch 1, worin die RNA ein pA:pU-5-mer ist.

6. RNA zur Verwendung in einem Verfahren der Anti-Leukämie-Therapie nach Anspruch 1, worin die RNA pA:pU mit Oligomeren von weniger als 10-mer-Länge ist.

7. RNA zur Verwendung in einem Verfahren der Anti-Leukämie-Therapie nach einem der vorangegangenen Ansprüche, worin die RNA pA:pU ist.

8. RNA zur Verwendung in einem Verfahren der Anti-Leukämie-Therapie nach einem der Ansprüche 1 bis 4, worin die RNA pA ist.

9. RNA zur Verwendung in einem Verfahren der Anti-Leukämie-Therapie nach einem der Ansprüche 1 bis 8, worin die Leukämiezellen T-Zell-Leukämiezellen oder menschliche Monozytenleukämiezellen sind.

10. RNA zur Verwendung in einem Verfahren der Anti-Leukämie-Therapie nach einem der Ansprüche 1 bis 9, worin die RNA mittels topischer Verabreichung, systemischer Verabreichung oder direkter Injektion in einen Tumor zu verabreichen ist.

11. RNA zur Verwendung in einem Verfahren der Anti-Leukämie-Therapie nach Anspruch 10, worin die Zusammensetzung eine Dosierung von pA oder pA:pU im Bereich von 1 bis 999 µg/kg umfasst.

12. RNA zur Verwendung in einem Verfahren der Anti-Leukämie-Therapie nach Anspruch 11, worin die Zusammensetzung eine Dosierung von pA oder pA:pU im Bereich von 1 bis 500 µg/kg umfasst.

13. RNA zur Verwendung in einem Verfahren der Anti-Leukämie-Therapie nach einem der vorangegangenen Ansprüche, worin der Patient ein Mensch ist.

14. Verwendung von RNA zur Herstellung einer Zusammensetzung zur Verwendung in einem Verfahren der Anti-Leukämie-Therapie, wobei das Verfahren das Induzieren von nicht apoptotischem Zelltod bei Leukämiezellen bei einem Patienten umfasst, worin die RNA pA:pU mit einem Molekulargewicht von 10 kDa oder weniger ist oder worin die RNA pA mit einem Molekulargewicht von 10 kDa oder weniger ist.

15. Verwendung nach Anspruch 14, worin die RNA erhöhte Zytokinproduktion von TNFα induziert.

16. Verwendung nach Anspruch 14, worin die RNA ein Molekulargewicht zwischen 1 kDa und 10 kDa aufweist.

17. Verwendung nach Anspruch 16, worin die RNA ein Molekulargewicht zwischen 1 kDa und 5 kDa aufweist.

18. Verwendung nach Anspruch 14, worin die RNA ein pA:pU-5-mer ist.

19. Verwendung nach Anspruch 14, worin die RNA pA:pU mit Oligomeren von weniger als 10-mer-Länge ist.

20. Verwendung nach einem der Ansprüche 14 bis 19, worin die RNA pA:pU ist.

21. Verwendung nach einem der Ansprüche 14 bis 17, worin die RNA pA ist.

22. Verwendung nach einem der Ansprüche 14 bis 21, worin die Leukämiezellen T-Zell-Leukämiezellen oder menschliche Monozytenleukämiezellen sind.

23. Verwendung nach einem der vorangegangenen Ansprüche, worin der Patient ein Mensch ist.

## Revendications

1. ARN pour utilisation dans une méthode de thérapie anti-leucémie, la méthode impliquant l'induction d'une mort cellulaire non-apoptotique dans des cellules leucémiques, où l'ARN est pA-pU ayant un poids moléculaire de 10 kDa ou moins, ou bien où l'ARN est pA ayant un poids moléculaire de 10 kDa ou moins.

2. ARN pour utilisation dans une méthode de thérapie anti-leucémie selon la revendication 1, où l'ARN induit une production de cytokine accrue de TNF-alpha.

3. ARN pour utilisation dans une méthode de thérapie anti-leucémie selon la revendication 1, où l'ARN a un poids moléculaire compris entre 1kda et 5kDa.

4. ARN pour utilisation dans une méthode de thérapie anti-leucémie selon la revendication 3, où l'ARN a un poids moléculaire compris entre 1kda et 5kDa.

5. ARN pour utilisation dans une méthode de thérapie anti-leucémie selon la revendication 1, où l'ARN est un pA:pU 5-mère.

6. ARN pour utilisation selon la revendication 1, où l'ARN est pA:pU ayant des oligomères inférieurs à une longueur de 10-mer.

7. ARN pour utilisation selon l'une quelconque des revendications précédentes, où l'ARN est pA:pU.

8. ARN pour utilisation dans une méthode de thérapie anti-leucémie selon l'une quelconque des revendications 1 à 4, où l'ARN est pA.

9. ARN pour utilisation selon l'une quelconque des revendications 1 à 8, où les cellules leucémiques sont des cellules leucémiques de cellules T ou des cellules leucémiques monocycliques humaines.

10. ARN pour utilisation selon l'une quelconque des revendications 1 à 9, où l'ARN doit être administrée par administration topique, administration systémique ou par injection directe dans une tumeur.

11. ARN pour utilisation dans une méthode de thérapie anti-leucémie selon la revendication 10, où la composition comprend un dosage de pA ou pA:pU dans la plage de 1-999µg/kg.

12. ARN pour utilisation dans une méthode de thérapie anti-leucémie selon la revendication 11, où la composition comprend un dosage de pA ou pA:pU dans la plage de 1-500µg/kg.

13. ARN pour utilisation dans une méthode de thérapie anti-leucémie selon l'une quelconque des revendications précédentes, où le patient est un être humain.

14. Utilisation de l'ARN dans la fabrication d'une composition pour utilisation dans une méthode de thérapie anti-leucémie, la méthode impliquant l'induction d'une mort cellulaire non-apoptotique dans des cellules leucémiques chez un patient, où l'ARN est pA:pU d'un poids moléculaire de 10kDa ou moins, ou bien où l'ARN est pA d'un poids moléculaire de 10kDa ou moins.

15. Utilisation selon la revendication 14, où l'ARN induit une plus grande production de cytokine de TNF-alpha.

16. Utilisation selon la revendication 14, où l'ARN a un poids moléculaire compris entre 1kda et 10kDa.

17. Utilisation selon la revendication 16, où l'ARN a un poids moléculaire compris entre 1kda et 5kDa.

18. Utilisation selon la revendication 14, où l'ARN est un pA:pU 5-mère.

19. Utilisation selon la revendication 14, où l'ARN est pA:pU ayant des oligomères inférieurs à une longueur de 10 mère.

20. Utilisation selon l'une quelconque des revendications 14 à 19, où l'ARN est pA:pU.

21. Utilisation selon l'une quelconque des revendications 14 à 17, où l'ARN est pA.

22. Utilisation selon l'une quelconque des revendications 14 à 21, où les cellules leucémiques sont des cellules leucémiques de cellules T ou des cellules leucémiques monocycliques humaines.

23. Utilisation selon l'une quelconque des revendications précédentes, où le patient est un être humain.
